(19) Europäisches Patentamt
European Patent Office
Office européen des brevets

(11) Veröffentlichungsnummer: **0 049 494**
**B1**

(12) # EUROPÄISCHE PATENTSCHRIFT

(45) Veröffentlichungstag der Patentschrift: **17.04.85**

(51) Int. Cl.⁴: **C 07 D 473/08,** A 61 K 31/52

(21) Anmeldenummer: **81107855.9**

(22) Anmeldetag: **02.10.81**

(54) **Ester von 7-Hydroxylalkyl-1,3-dimethylxanthinen, Verfahren zu ihrer Herstellung und ihre Verwendung als lipidsenkende Mittel.**

(30) Priorität: **03.10.80 DE 3037391**

(43) Veröffentlichungstag der Anmeldung: **14.04.82 Patentblatt 82/15**

(45) Bekanntmachung des Hinweises auf die Patenterteilung: **17.04.85 Patentblatt 85/16**

(84) Benannte Vertragsstaaten: **AT BE CH DE FR GB IT LI NL SE**

(56) Entgegenhaltungen:
**CH - A - 348 159**
**DE - A - 2 308 826**
**DE - A - 2 461 069**
**FR - M - 814**
**NL - C - 99 186**

**ARZNEIMITTELFORSCHUNG, Band 25, Nr. 11, November 1975 AULENDORF (DE) G. METZ et al.: "Hypolipemic Activity of Clofibrate-Related Compounds", Seiten 1686-1692**
**ARZNEIMITTELFORSCHUNG, Band 30, Nr. 11b, November 1980 AULENDORF (DE) G. METZ et al.: "Zur Chemie der Phenoxyessigsäureester von Oxylakyltheophyllinen und 1-(Theophyllin-7-yl)-äthyl-2-(2-(p-chlorphenoxy)-2-methyl-propionat) (Etofyllinclofibrat), einem neuen**

(73) Patentinhaber: **King Consult GmbH, Mittelstrasse 54, D-5000 Köln 1 (DE)**

(72) Erfinder: **Wieland, Ernst, Dr., Hauptstrasse 72, D-5024 Pulheim 3 (DE)**
Erfinder: **Schröder, Hermann, Prof. Dr.Dr., Adenauer Allee 355, D-5100 Aachen (DE)**

(74) Vertreter: **Werner, Hans-Karsten, Dr. et al, Deichmannhaus am Hauptbahnhof, D-5000 Köln 1 (DE)**

(56) Entgegenhaltungen: (Fortsetzung)
**Antilipämikum", Seiten 2014-2019**

**Die Akte enthält technische Angaben, die nach dem Eingang der Anmeldung eingereicht wurden und die nicht in dieser Patentschrift enthalten sind.**

**EP 0 049 494 B1**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Ester von 7-Hydroxyalkyl-1,3-dimethylxanthinen, von denen einige bereits in der Literatur beschrieben sind, andere jedoch noch neu sind, ein verbessertes Verfahren zur Herstellung dieser Substanzen und schließlich diese Stoffe zur lipidsenkenden Verwendung allein oder in Kombination als lipidsenkende Mittel.

$\beta$-Chlorphenoxyisobutylsäure (Clofibrinsäure) (J. M. Thorp & S. Waring: Nature 194, 948 (1962)) und Nicotinsäure (L. A. Carlson, L. Örö: J. Atheroscler, Res. 8, 667 (1968)) sowie die vor kurzem beschriebene 4-Benzyloxybenzoesäure (K. H. Baggeley et al.: J. med. Chem. 20, 1388 (1977)); G. De Vries et al.: J. med. Chem. 19, 446 (1976)) haben lipidsenkende Eigenschaften und wurden in die Therapie der Hyperlipoproteinämien eingeführt.

Die Monotherapie mit den erwähnten Verbindungen verlangt eine hohe Dosierung, die sich der Verträglichkeitsgrenze nähert und zu einer Reihe unerwünschter Nebenwirkungen, dabei aber oft nicht zum Erfolg führt.

Bekanntlich hemmen auch Xanthine und deren Derivate, wie z. B. die Hydroxyalkyl-dimethylxanthine, die magnesiumabhängigen Phosphodiesterase vom cyclischen Adenosino 3',5'-Monophosphat (c-3',5'-AMP). Dadurch kommt es zur Aktivierung von Lipasen und zum Zerfall der Triglyceride, was eine Senkung der Plasmalipide zur Folge hat (J. A. Beavo et al.: Molec. Pharmacol. 6, 597 (1970)).

Es sind auch schon Clofibrat ähnliche Verbindungen synthetisiert und geprüft worden, vgl. Arzneimittelforschung 25, 1975, Seite 1686—1695 und DE-A 2 308 826. Die beste dieser Substanzen, 1-(Theophyllin-7-yl)-äthyl-2-[2-(p-chlorphenoxy)-2-methylpropionat] mit der Kurzbezeichnung Etofyllinclofibrat und der Prüfnummer ML 1024 wurde unter der Bezeichnung Duolip$^{R}$ in die Therapie eingeführt.

Aus der AT-A-220 157, der CH-A-348 159 und der FR-M-814 sind Nikotinsäureester von 7-Hydroxyalkyl-1,3-dimehtylxanthinen bekannt geworden, die jedoch als periphere Kreislaufmittel beschrieben wurden. Eine Einführung in die Therapie haben diese Substanzen jedoch nicht gefunden. Weitere Derivate des Clofibrats sind bekannt aus der DE-A-2 461 069, die aber ebenfalls keinen Eingang in die Therapie gefunden haben.

Es wurde jetzt gefunden, daß Ester von 7-Hydroxyalkyl-1,3-dimethylxanthinen der allgemeinen Formel I

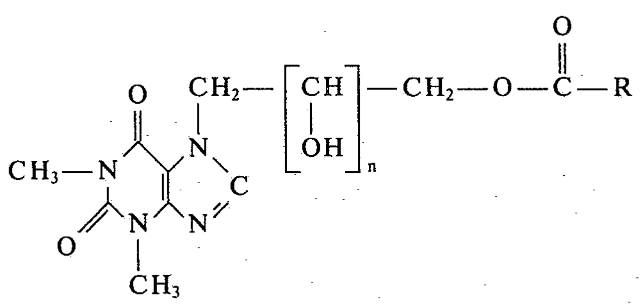

in der n 0 oder 1 und R die Reste

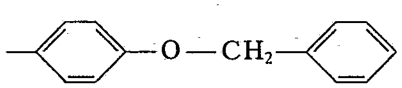 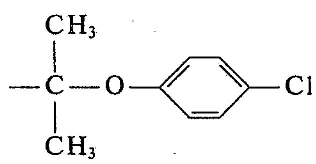 oder 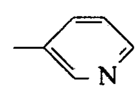

bei peroraler Gabe eine hervorragende lipidsenkende Wirkung aufweisen, dabei jedoch wesentlich besser verträglich sind als die bisher in die Therapie eingeführten lipidsenkenden Mittel. Besonders vorteilhaft erweist sich die Kombination einer oder mehrerer dieser Verbindungen, da sie in synergistischer Weise die lipidsenkende Wirkung erhöhen und daher in besonders niedriger Dosierung eingesetzt werden können. Hierbei spielt offensichtlich auch die bessere Resorbierbarkeit eine entscheidende Rolle. Hohe Resorbierbarkeit ermöglicht nicht nur eine niedrigere Dosierung, sondern auch eine Erhöhung der Sicherheit der Therapie, da die dem Körper zur Verfügung gestellten Mengen bei hoher Resorbierbarkeit wesentlich geringeren Schwankungen unterliegen als bei schlecht resorbierbaren Substanzen.

Die Substanzen wirken einerseits aus sich heraus, zum anderen entstehen beim hydrolytischen Abbau Clofibrinsäure, Nikotinsäure, Benzyloxybenzoesäure und Xanthinderivate, die in unterschiedlichster Weise obendrein lipidsenkend wirken und dadurch die Gesamtwirkung entsprechender Arzneimittel erhöhen.

Insbesondere in Kombination wirken die Substanzen sowohl auf den Cholesterin- als auch auf den Triglyceridspiegel, so daß sie für die Hyperlipoproteinämie der Typen II b, IV und V wirksam eingesetzt werden können.

Einige der bevorzugten erfindungsgemäß einsetzbaren 7-Hydroxyalkyl-1,3-dimethylxanthine sind in der Literatur noch nicht beschrieben. Insbesondere handelt es sich dabei um die Substanzen der Formel I,

in der n die Zahlen 0 oder 1 und R den Rest

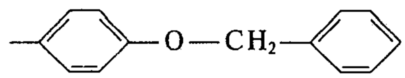

und für den Fall, daß n gleich 1 ist, auch den Rest

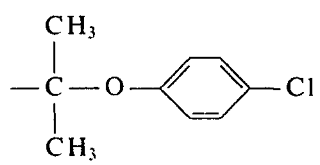

darstellt.

Diese neuen, aber auch die bereits in der Literatur beschriebenen erfindungsgemäß verwendbaren Substanzen können nach einem neuen, überraschend gut und glatt verlaufenden Verfahren hergestellt werden. Bisher wurden diese Substanzen durch Umsetzung von Carbonsäurechloriden, Carbonsäureanhydriden mit den entsprechenden Hydroxyalkylxanthinen hergestellt. In einigen Fällen ist es auch gelungen, die Hydroxyalkylxanthine mit den Carbonsäuren unter azeotropen Bedingungen zu destillieren und dabei die entsprechenden Ester zu erhalten. Diese Verfahren verliefen im allgemeinen nur mit schlechten Ausbeuten und der Bildung von unerwünschten Nebenprodukten, da nicht nur die endständige OH-Gruppe, sondern auch die mittelständige OH-Gruppe sich an der Reaktion beteiligte.

Es wurde jetzt gefunden, daß überraschenderweise Verbindungen der allgemeinen Formel II

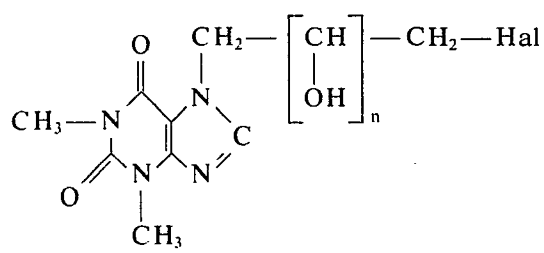

(II)

in denen n die Zahlen 0 oder 1 darstellt und Hal für ein Chlor- oder Bromatom steht beim Erhitzen mit dem Anion der entsprechenden Carbonsäuren (III)

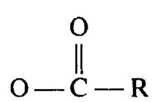

in einem Lösungsmittel in Anwesenheit eines tertiären Amins auf 80 bis 200°C in glatter Reaktion und hoher Ausbeute und Reinheit die gewünschten Ester bilden. Dieses Ergebnis ist außerordentlich überraschend, da bekannt war, daß Halogenalkyl und Hydroxyhalogenxanthine in basischem Medium unter Halogenwasserstoffabspaltung die entsprechenden Vinyl- bzw. Epoxypropylderivate bilden. Es erfolgt somit eine Eliminierung und keine Substitutionsreaktion. Als tertiäre Amine eignen sich besonders das N,N-Dimethylanilin, das N-Methylmorpholin, N-Äthylpiperidin oder Triäthylamin. Als Lösungsmittel eignen sich besonders das Methoxyäthanol, 1,2-Dimethoxyäthan oder Dimethylformamid. Die Reaktion wird bei 80 bis 200°C, vorzugsweise 95 bis 180°C durchgeführt. Die Reaktionspartner werden im allgemeinen in äquimolaren Mengen eingesetzt. Vorzugsweise wird ein geringer Überschuß der Carbonsäure eingesetzt. Dieser Überschuß sollte jedoch nicht mehr als 1 Mol betragen. Besonders bewährt haben sich Überschüsse von 0 bis 0,1 Mol.

Die Aufarbeitung erfolgt in besonders einfacher Weise, da nach dem Abkühlen auf Raumtemperatur im allgemeinen die Hydrohalogenide der tertiären Amine ausfallen und leicht abgetrennt werden können. Die gewünschten Ester können in üblicher Weise gereinigt werden und fallen von vornherein in hoher Ausbeute und guter Reinheit an.

In den nachfolgenden Beispielen wird die Erfindung näher erläutert.

### Beispiel 1

7-β-Hydroxy-γ-(p-chlorphenoxy-isobutyroyloxy)-propyl-1,3-dimethylxanthin

In 400 ml Dimethylformamid (DMF) wird 232 g (1,1 Mol) p-Chlorphenoxyisobutersäure unter Zusatz von 148 ml (1,1 Mol) Triäthylamin bei Zimmertemperatur gelöst und nach der Zugabe von 273 g (1,0 Mol) 7-β-Hydroxy-γ-Chlorpropyl-1,3-dimethylxanthin 6–8 Stunden unter dem Rückfluß auf dem kochenden Wasserbad gerührt. Nach der Abkühlung zur Raumtemperatur scheidet sich Triäthylaminhydrochlorid ab. Die Kristalle werden abgesaugt und mit 3 × 50 ml Aceton durchgewaschen. Die vereinigten Mutterlaugen werden bei −10°C abgekühlt und der Niederschlag von rohem Ester abgetrennt, zuerst mit Aceton, dann mit Wasser chloridfrei gewaschen.

Aus der Mutterlauge nach dem Einengen (Wasserstrahlpumpe) erhält man zusätzliche Mengen vom Reaktionsprodukt.

Das Reaktionsprodukt kann auch durch Abdestillieren ca. 200 ml DMF in Vakuum und Zugabe von ca. der 10fachen Menge von Aceton nach längerem Abkühlen bei −5°C isoliert und durch Waschen mit Wasser von Begleitsubstanzen gereinigt werden.

Die Gesamtausbeute beträgt 360–410 g (80–90 %) Schmp. 97–102°C.

Nach der Umkristallisierung aus Methanol oder vorteilhafter Methanol-Aceton (2 : 1) erhält man reines 7-β-Hydroxy-γ-(p-chlorphenoxy-isobutyroyloxy)propyl-1,3-dimethylxanthin von Schmp. 105–107°C.

$C_{20}H_{23}O_6N_4Cl$ (450,5):

| | | | |
|---|---|---|---|
| Ber. | 53,58% C, | 5,15% H, | 12,13% N. |
| Gef. | 53,28% C, | 4,98% H, | 12,09% N. |

### Beispiel 2

7-(β-Hydroxy-γ-nikotinoyloxy)propyl-1,3-dimethylxanthin

Zu einer Lösung von 217,6 g (0,8 Mol) 7-β-Hydroxy-γ-Chlorpropyl-1,3-dimethylxanthin in 325 ml Dimethylformamid (DMF) und 88,0 (0,88 Mol) Triäthylamin (über KOH getrocknet) wird 108,2 g (0,88 Mol) Nicotinsäure zugegeben und unter Rühren 3 Std. unter Rückfluß gekocht. Die Reaktionsmischung wird im Kühlschrank abgekühlt und das ausgeschiedene rohe Reaktionsprodukt abfiltriert.

Das Produkt wird in 300 ml Wasser eingemischt, aufgekocht, abgekühlt (5 Std. bei 0°C), abfiltriert und auf dem Filter chloridfrei mit Wasser gewaschen. Man erhält 200–204 g des rohen 7-β-Hydroxy-γ-nikotinoyloxy)propyl-1,3-dimethylxanthin.

Aus der DMF-Mutterlauge, nach dem Aufkochen mit Aktivkohle kann man durch Vakuumdestillation (Wasserstrahlpumpe) ca. 70% des DMF zurückgewinnen. Der Destillationsrückstand nach Zugabe von ca. 100 ml Wasser und üblicher Aufarbeitung ergibt noch weitere Mengen des Produktes (ca. 6–10 g).

Gesamtausbeute 200–210 g (72–74%), Schmp. 200–205°C. Nach der Umkristallisierung aus Methanol oder Methoxyethanol Schmp. 204–207°C.

$C_{16}H_{17}O_5N_5$ (359,2):

| | | | |
|---|---|---|---|
| Ber. | 53,53% C, | 4,77% H, | 19,51% N. |
| Gef. | 53,45% C, | 4,54% H, | 19,24% N. |

Die Löslichkeit bei Siedetemperatur: 6,5% in Äthanol, 5% in Methoxyethanol.

### Beispiel 3

7-β-Hydroxy-γ-(4-Benzyloxybenzoyloxy)-propyl-1,3-dimethylxanthin

Aus 125 g (0,55 Mol) 4-Benzyloxybenzoesäure 75 ml Triäthylamin und 165 g (0,5 Mol) 7-β-Hydroxy-γ-Chlorpropyl-1,3-dimethylxanthin wird gemäß Beispiel 2 7β-Hydroxy-γ-(4-Benzyloxybenzoyloxy)propyl-1,3-dimethylxanthin in einer Ausbeute von 90–95% erhalten. Schmp. 149–156°C, nach der Umkristallisierung aus Äthanol Schmp. 159–161°C.

$C_{25}H_{25}O_6N_4$ (477,5):

| | | | |
|---|---|---|---|
| Ber. | 62,90% C, | 5,28% H, | 11,74% N. |
| Gef. | 62,59% C, | 5,38% H, | 11,61% N. |

## Beispiel 4

### 7-β-(4-Benzyloxybenzoyloxy)äthyl-1,3-dimethylxanthin

Die Verbindung wurde gemäß Beispiel 3 durch Reaktion von 14,35 g (0,05 Mol) 7-β-Bromäthyl-1,3-dimethylxanthin (oder äquivalente Menge 7-β-Chloräthyl-1,3-dimethylxanthin 12,5 g (0,055 Mol) 4-Benzyloxybenzoesäure und 7,5 ml Triäthylamin in einer Ausbeute von ca. 16—17 g erhalten. Das rohe Produkt wird aus Äthanol umkristallisiert, Schmp. 145—147°C.

$C_{23}H_{22}O_5N_4$ (435,5):

| | | | |
|---|---|---|---|
| Ber. | 63,58%C, | 5,11%H, | 12,89%N. |
| Gef. | 63,73%C, | 5,15%H, | 12,69%N. |

Die erfindungsgemäßen Arzneimittel sind pharmazeutische Zubereitungen, die neben nicht-toxischen, inerten, pharmazeutisch geeigneten Trägerstoffen einen oder mehrere der oben erwähnten Wirkstoffe enthalten, oder die aus einem oder mehreren dieser Wirkstoffe bestehen.

Diese pharmazeutischen Zubereitungen liegen meist in Dosierungseinheiten vor. Dies bedeutet, daß die Zubereitungen in Form einzelner Teile, z. B. Tabletten, Dragees, Kapseln, Pillen vorliegen, deren Wirkstoffgehalt einem Bruchteil oder einem Vielfachen einer Einzeldosis entsprechen. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste Verdünnungsmittel, Füllstoffe und Formulierungshilfsmittel jeder Art zu verstehen. Zu ihnen gehören Füll- und Streckmittel wie Stärken, Milchzucker, Rohrzucker, Glucose, Manid und Kieselsäure, Bindemittel wie Carboxymethylcellulose, Alginate, Gelatine, Polyvinylpyrrilidon, Feuchthaltemittel wie Glycerin, Sprengmittel wie Agar, Calciumcarbonat und Natriumdicarbonat, Lösungsverzögerer, z. B. Paraffin und Resorptionsbeschleuniger, z. B. quaternäre Ammoniumverbindungen, Adsorptionsmittel wie Kaolin und Bentonid und Gleitmittel wie Talkum, Calcium und Magnesiumstearat und feste Polyäthylenglykole sowie Gemische der oben aufgeführten Stoffe.

Der oder die Wirkstoffe können ggf. mit einem oder mehreren der oben genannten Trägerstoffe auch in mikroverkapselter Form vorliegen zur Erzielung eines Retardeffektes.

Die genannten Formulierungsformen können auch Färbemittel, Konservierungsstoffe sowie geruchs- und geschmacksverbessernde Zusätze, z. B. Pfefferminzöl und Eukalyptusöl sowie Süßmittel, z. B. Saccharin enthalten.

Die therapeutisch wirksamen Verbindungen sollen in den oben aufgeführten pharmazeutischen Zubereitungen vorzugsweise in einer Konzentration von 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die erfindungsgemäßen Wirkstoffe werden in Gesamtmengen von etwa 1000 mg bis 2000 mg, vorzugsweise etwa 1500 mg je 24 Stunden, ggf. in Form mehrerer Einzelgaben zur Erzielung der gewünschten Ergebnisse verabreicht. Eine Einzelgabe erhält den oder die erfindungsgemäßen Wirkstoffe, vorzugsweise in Mengen von etwa 300 mg bis etwa 700 mg, insbesondere ungefähr 500 mg/Dosis. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als den oben genannten Mengen Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

**Patentansprüche**

1. Ester von 7-Hydroxyalkyl-1,3-dimethylxanthinen der allgemeinen Formel I

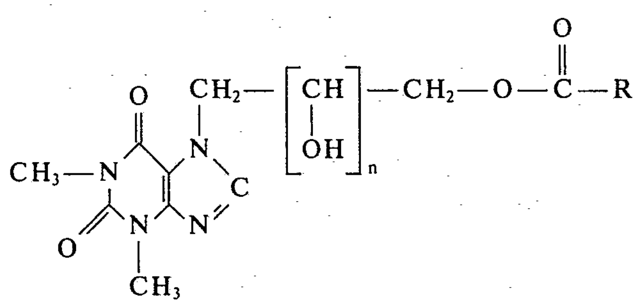

(I)

in der n die Zahlen 0 oder 1 und R den Rest

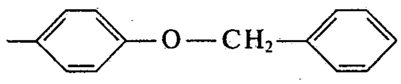

und für den Fall, daß n gleich 1 ist, auch den Rest

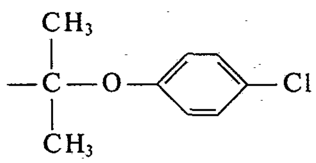

darstellt.

2. Verfahren zur Herstellung von Estern von 7-Hydroxyalkyl-1,3-dimethylxanthinen der allgemeinen Formel I

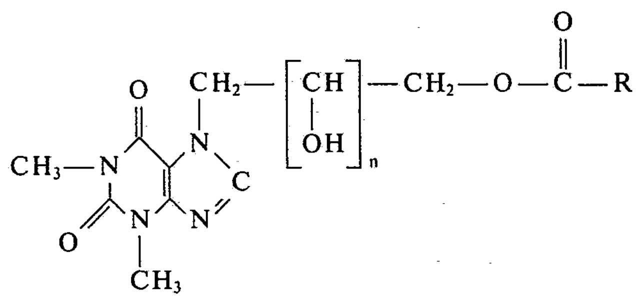

(I)

in der n 0 oder 1 und R die Reste

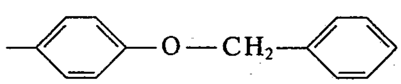 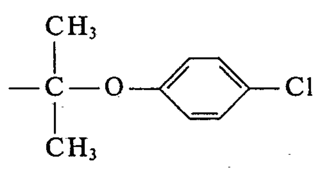 oder 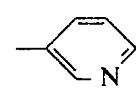

darstellt, dadurch gekennzeichnet, daß man Verbindungen der allgemeinen Formel II

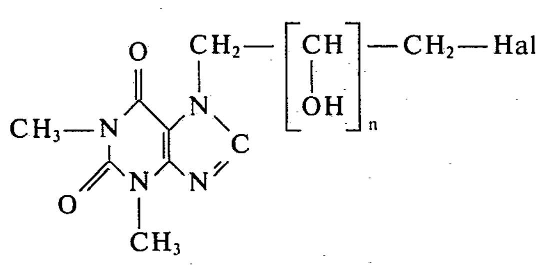

(II)

in denen n die Zahlen 0 oder 1 darstellt und Hal für ein Chlor- oder Bromatom steht, mit Verbindungen der allgemeinen Formel III

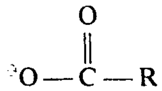

(III)

in der R die oben angegebene Bedeutung hat in einem Lösungsmittel in Anwesenheit eines tertiären Amins auf 80–200°C, vorzugsweise 95 bis 180°C erhitzt.

3. Verfahren nach Anspruch 2, dadurch gekennzeichnet, daß man als tertiäre Amine N,N-Dimethylanilin, N-Methylmorpholin, N-Äthylpiperidin oder Triäthylamin einsetzt.

4. Verfahren nach Ansprüchen 2 und 3, dadurch gekennzeichnet, daß man als Lösungsmittel Methoxyäthanol, 1,2-Dimethoxyäthan oder Dimethylformamid einsetzt.

5. Lipidsenkende Arzneimittel enthaltend mindestens eine Verbindung der Formel I, in der n 0 oder 1 und R den Rest

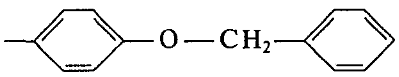

und für den Fall, daß n gleich 1 ist, auch den Rest

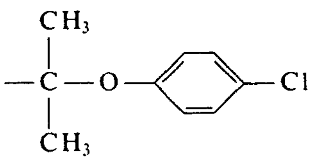

darstellt, und übliche Hilfs- und Trägerstoffe.

6. Lipidsenkendes synergistisches Kombinationsmittel nach Anspruch 5, dadurch gekennzeichnet, daß sie 7-β-Hydroxy-γ-(p-chlorphenoxy-isobutyroyloxy)propyl-1,3-dimethylxanthin in Kombination mit 7-β-Hydroxy-γ-(nikotinoyloxy)propyl-1,3-dimethylxanthin und/oder mit 7-β-Hydroxy-γ-(4'benzyloxybenzoyloxy)propyl-1,3-dimethylxanthin oder 7-β-(4'-benzyloxybenzoyloxy)äthyl-1,3-dimethylxanthin enthalten.

**Claims**

1. An ester of a 7-hydroxyalkyl-1,3-dimethylxanthine having the general formula I

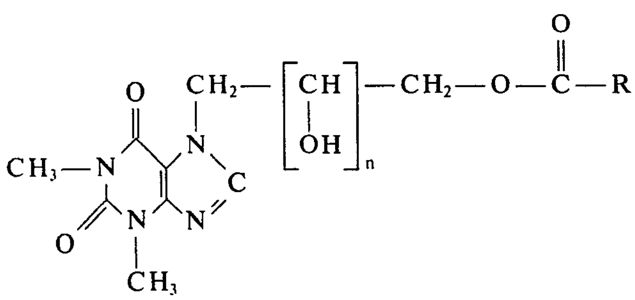

(I)

wherein n represents the numbers 0 or 1 and R represents the residue

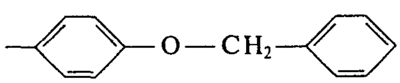

and, in the case that n is equal to 1, also the residue

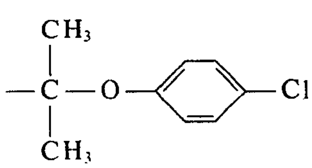

2. A process for preparing an ester of a 7-hydroxyalkyl-1,3-dimethylxanthine having the general formula I

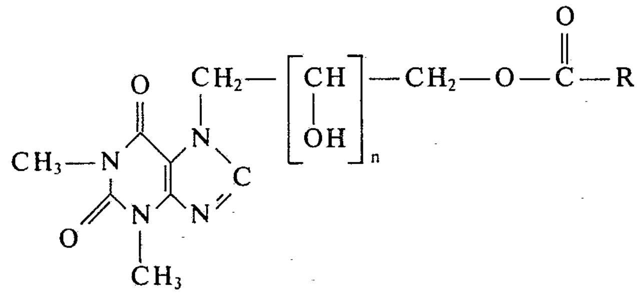

(I)

wherein n represents the numbers 0 or 1 and R represents the residues

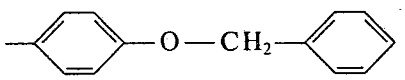 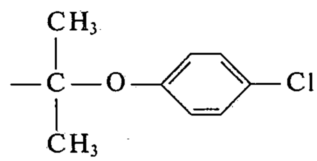 or 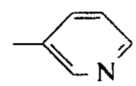

characterized in that a compound having the general formula II

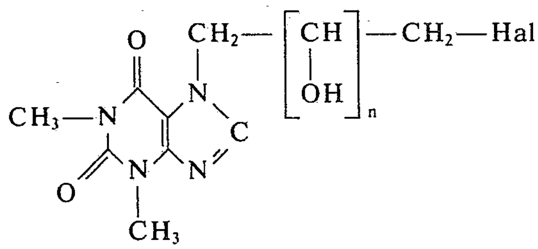

(II)

wherein n represents the numbers 0 or 1 and Hal represents a chlorine or bromine atom, are heated at 80°C–200°C, preferably at 95°C to 180°C, with a compound having the general formula III

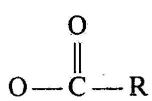

(III)

wherein R is as defined above in a solvent in the presence of a tertiary amine.

3. The process according to claim 2, characterized in that N,N-dimethylaniline, N-methylmorpholine, N-ethylpiperidine or triethylamine are used as the tertiary amine.

4. The process according to claims 2 and 3, characterized in that methoxyethanol, 1,2-dimethoxyethane or dimethylformamide are used as the solvent.

5. A lipid-lowering medicament, containing at least on compound having the formula I wherein n represents 0 or 1 and R represents the residue

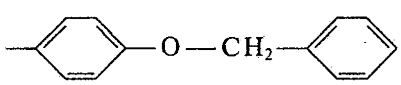

and, in the case that n is equal to 1, also the residue

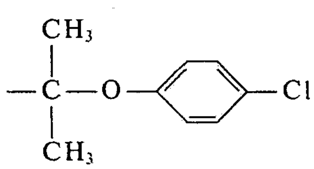

and conventional excipients and carriers.

6. A lipid-lowering synergistic combination medicament according to claim 5, characterized in that it contains 7-β-hydroxy-γ-(p-chlorophenoxy-isobutyroyloxy)propyl-1,3-dimethylxanthine in combination with 7-β-hydroxy-γ-(nicotinoyloxy)propyl-1,3-dimethylxanthine and/or with 7-β-hydroxy-γ- (4'-benzyloxybenzoyloxy)propyl-1,3-dimethylxanthine or 7-β-hydroxy-γ-(4'-benzyloxybenzoyloxy)ethyl-1,3-dimethylxanthine.

**Revendications**

1. Esters de 7-hydroxyalkyl-1,3-diméthylxanthines de formule générale I

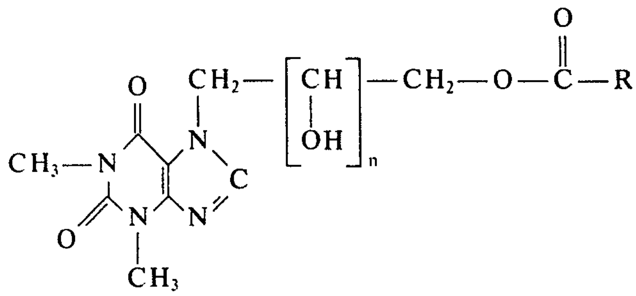

(I)

dans laquelle n représente les nombres 0 ou 1 et R le reste

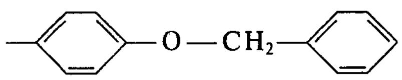

et, au cas où n est égal à l, également le reste

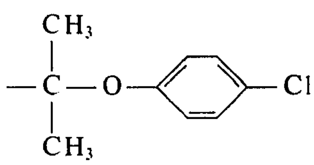

2. Procédé pour la préparation d'esters de 7-hydroxyalkyl-1,3-diméthylxanthines de formule générale I

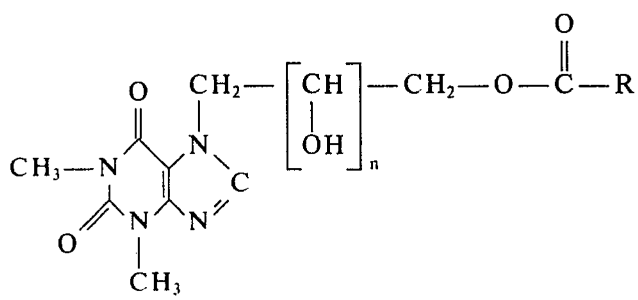

(I)

dans laquelle n représente 0 ou 1 et R les restes

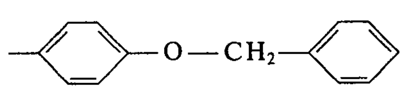 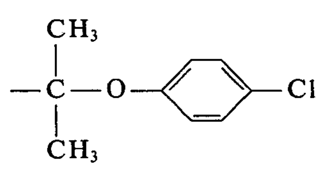 ou 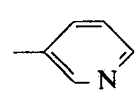

caractérisé en ce que l'on chauffe à 80–200℃, de préférence de 95 à 180℃, dans un solvant en présence d'une amine tertiaire, des composés de formule générale II

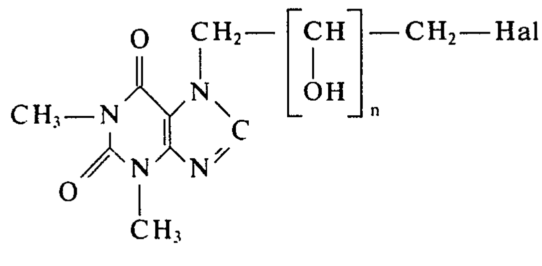

(II)

dans laquelle n représente le nombre 0 ou 1 et Hal est un atome de chlore ou de brome, avec des composés de formule générale III

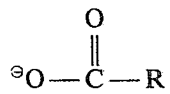 (III)

dans laquelle R a la signification indiquée ci-dessus.

3. Procédé selon la revendication 2, caractérisé en ce que l'on utilise comme amine tertiaire la N,N-diméthylaniline, la N'-méthylmorpholine, la N-éthylpipéridine ou la triéthylamine.

4. Procédé selon les revendications 2 et 3, caractérisé en ce que l'on utilise comme solvant le méthoxyéthanol, le 1,2-diméthoxyéthane ou le diméthylformamide.

5. Médicament de diminution des lipides renfermant au moins un composé de formule I, dans laquelle n représente 0 ou 1 et R le reste

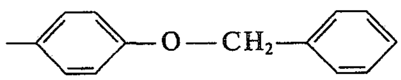

et, dans le cas où n est égal à I, également le reste

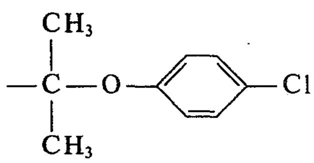

et des adjuvants et supports usuels.

6. Agents de combinaison synergétique de diminution des lipides selon la revendication 5, caractérisés en ce qu'ils renferment de la 7-$\beta$-hydroxy-$\gamma$-(p-chlorophénoxy-isobutyroyloxy)propyl-1,3-diméthylxanthine en combinaison avec de la 7-$\beta$-hydroxy-$\gamma$-(nicotinoyloxy)propyl-1,3-diméthylxanthine et/ou avec la 7-$\beta$-hydroxy-$\gamma$-(4'-benzyloxybenzoyloxy)propyl-1,3-diméthylxanthine ou 7-$\beta$-(4'-benzyloxybenzoyloxy)éthyl-1,3-diméthylxanthine.